# EUROPEAN PATENT SPECIFICATION

(11) **EP 3 881 872 B1**
(45) Date of publication and mention of the grant of the patent: **10.12.2025**
(21) Application number: 20164340.0
(22) Date of filing: 19.03.2020
(51) Int. Cl.: A61F 2/00, A61L 31/02, A61L 31/14, A61L 31/16, A61M 27/00

(54) **COMBINATION BIOACTIVE SILICATE MEDICINE CARRIER AND SHUNT**
KOMBINATION AUS EINEM BIOAKTIVEN SILIKAT-MEDIZINTRÄGER UND EINEM SHUNT
SUPPORT MÉDICAL ET DÉRIVATION COMBINÉS DE SILICATE BIOACTIF

(43) Date of publication of application: 22.09.2021
(73) Proprietor: Taiwan Fiber Optics, Inc., Taipei City 104 (TW)
(72) Inventor: LU, LUKE, San Diego, CA 2131 (US); TSENG, HSIAO SEN, 420 Taichung City (TW); LU, MICHELLE, San Diego, CA 92131 (US); LU, EMILY, San Diego, CA 92131 (US)
(74) Representative: Lang, Christian

(56) References cited:
- WO-A2-2008/035088
- US-A1- 2008 161 741
- US-A1- 2018 125 707
- US-A1- 2019 099 515
- EL-KADY ABEER M. ET AL: "Bioactive Glass Nanoparticles as a New Delivery System for Sustained 5-Fluorouracil Release: Characterization and Evaluation of Drug Release Mechanism", JOURNAL OF NANOMATERIALS, vol. 2015, 1 January 2015 (2015-01-01), US, pages 1 - 11, XP93038121, ISSN: 1687-4110, Retrieved from the Internet <URL:http://downloads.hindawi.com/journals/jnm/2015/839207.pdf> [retrieved on 20230405], DOI: 10.1155/2015/839207

## Description

### BACKGROUND OF THE INVENTION

### 1. Field of the Invention

The invention relates to shunts and more particularly to a combination bioactive silicate glass (e.g., bioactive glass) medicine carrier and shunt, as defined in the claims. The shunt being adapted to serve as a support of an eyeball, a stent of a blood vessel, or the like so as to lessen transplant rejection, the shunt being adapted to be bioresorbable in a predetermined period of time so as to solve problems of the transplanted tissue rejection and side effect, and the shunt being adapted to direct flow and store medicine.

### 2. Description of Related Art

Conventionally, after inserting a stent into the lumen of an anatomic vessel of a patient, the patient is required to swallow anti-inflammatory drugs or anti-scarring drugs to less transplant rejection. A large amount of medicine may be dissolved in blood which may circulate through blood vessels to heal a wound because the medicine is swallowed. However, the stent has a probability of 5-10% being blocked annually. Thus, replacement of the stent by operation is required. Unfortunately, there is risk in the operation.

Thus, the need for increasing the performance of an inserted stent in healing a wound exists.

Patent application publication US 2008/0161741 A1 discloses an ocular implant device that is insertable into either the anterior or posterior chamber of the eye to drain aqueous humor and/or to introduce medication. From patent application publication US 2019/0099515 A1 implant devices comprising an improved bone graft material, such as bioactive glass, are known.

### SUMMARY OF THE INVENTION

The invention provides a combination bioactive silicate medicine carrier and shunt having the features of claim 1.

Further embodiments are subject-matter of the dependent claims.

The combination bioactive silicate glass medicine carrier and shunt according to the invention comprises a shunt which is a micro capillary member made of bioactive silicate glass , the shunt including an insertion end at a first end; wherein the shunt further comprises an axial channel. The gaps gaps in the bioactive silicate glass, serve as at least one medicine storage for storing medicines.

The combination bioactive silicate glass medicine carrier and shunt according to the invention is further defined by the shunt including at least one direction check member disposed on an outer surface and a tapered insertion end at a first end; wherein gaps in the bioactive silicate glass define a plurality of tunnel-shaped medicine storages equally spaced around the axial channel for storing medicines.

The invention has the following advantages and benefits in comparison with the conventional art: The shunt is made of bioactive silicate glass (e.g., bioactive glass). After the shunt has been inserted into the human body, it is capable of minimizing transplant rejection. Further, the shunt can control amount of medicine dissolved in the human body as time evolves. Furthermore, different parts of the shunt are degraded as time evolves so that a desired effect can be carried out by effectively controlling time of discharging medicine. In addition, one or more kinds of medicine can be stored in the medicine storages. After the shunt has been inserted into a predetermined position of the human body, a small amount of medicine is sufficient to heal a wound since the medicine is discharged at a position proximate the location of the wound (or a target).

The above and other objects, features and advantages of the invention will become apparent from the following detailed description taken with the accompanying drawings.

### BRIEF DESCRIPTION OF THE DRAWINGS

FIG. 1 is a perspective view of a shunt (not part of the invention)
FIG. 2 is a perspective view of a shunt (not part of the invention) showing fiber bundle disposed through an axial channel;
FIG. 3 is a perspective view of a shunt according to the invention showing a tapered insertion end and a direction check member;
FIG. 4 is a longitudinal sectional view of FIG. 3;
FIG. 5 is a side elevation of FIG. 3;
FIG. 6 is a sectional view taken along line 6-6 of FIG. 5;
FIG. 7 is a cross-sectional view of a shunt according to a preferred embodiment of the invention;
FIG. 8 is a cross-sectional view of a shunt according to a preferred embodiment of the invention;
FIG. 9 is a side elevation of a shunt according to a preferred embodiment of the invention;
FIG. 10 is a sectional view taken along line 10-10 of FIG. 9;
FIG. 11 schematically depicts the shunt according to the invention inserted into a blood vessel as support;
FIG. 12 schematically depicts a shunt according to the invention provided in an eyeball for draining high pressure fluid; and
FIG. 13 is a detailed view of a circle of FIG. 12.

### DETAILED DESCRIPTION OF THE INVENTION

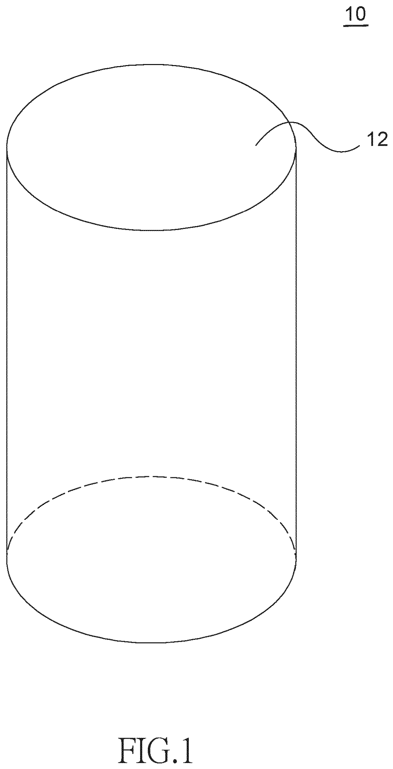

Referring to FIG. 1, a combination bioactive silicate glass (e.g., bioactive glass) medicine carrier and shunt (not part of the invention). The combination bioactive silicate glass (e.g., bioactive glass) medicine carrier and shunt comprises a shunt 10 which is a micro capillary member made of bioactive silicate glass (e.g., bioactive glass). The shunt 10 includes an insertion end 12 at one end. Gaps in the bioactive silicate glass (e.g., bioactive glass) are served as at least one storage (not shown) for storing medicines.

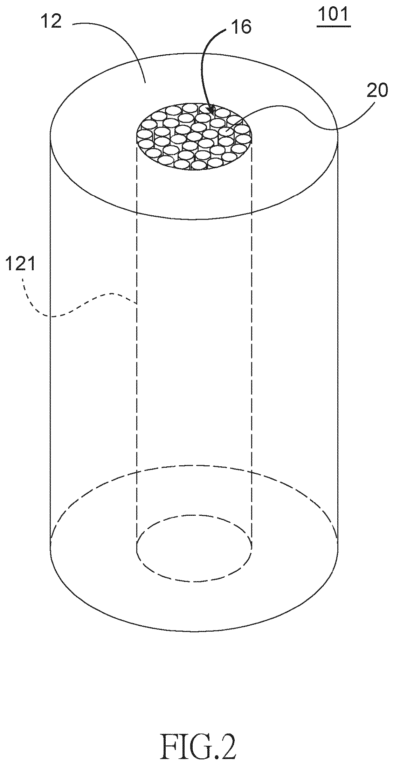

Referring to FIG. 2, a combination bioactive silicate glass (e.g., bioactive glass) medicine carrier and shunt (not part of the invention) is shown. The characteristics are substantially the same as that of the first figure except the following:
A plurality of fiber bundle 20 are provided through an axial channel 121 of a fiber bundle shunt 101. A gap among the fiber bundle 20 is defined as a medicine storage 16 for storing medicine. After the fiber bundle shunt 101 has been inserted into a predetermined position of the body, a small amount of medicine is sufficient to heal a wound since the medicine is discharged at a position proximate the location of the wound (or a target).

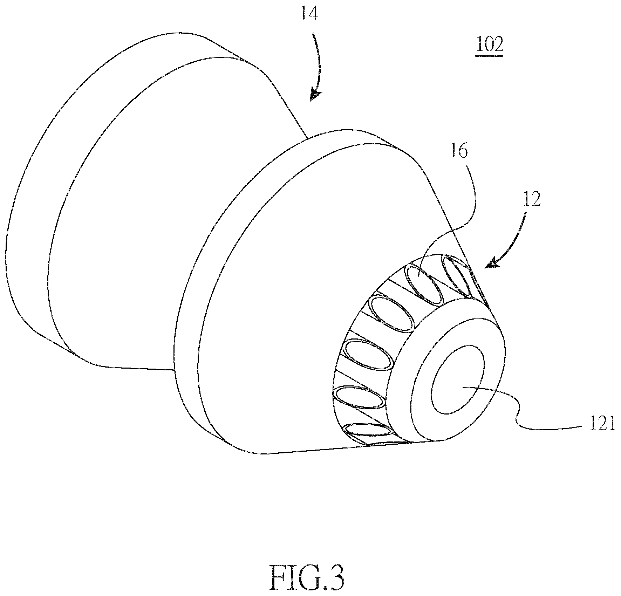

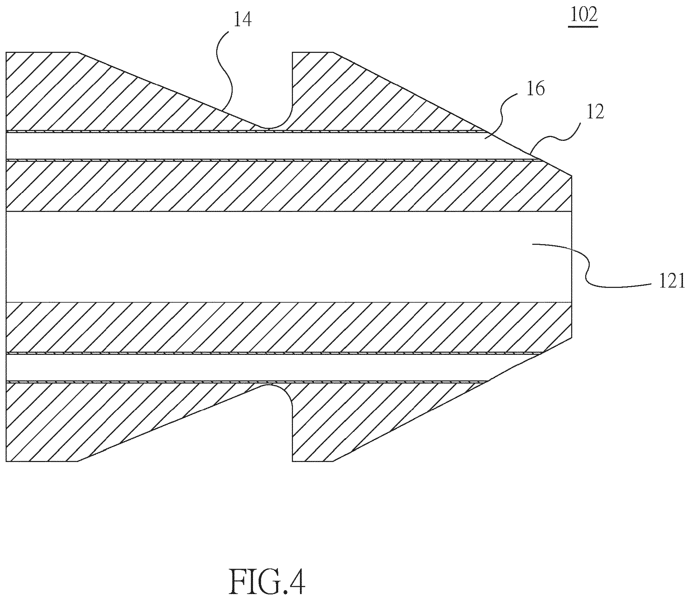

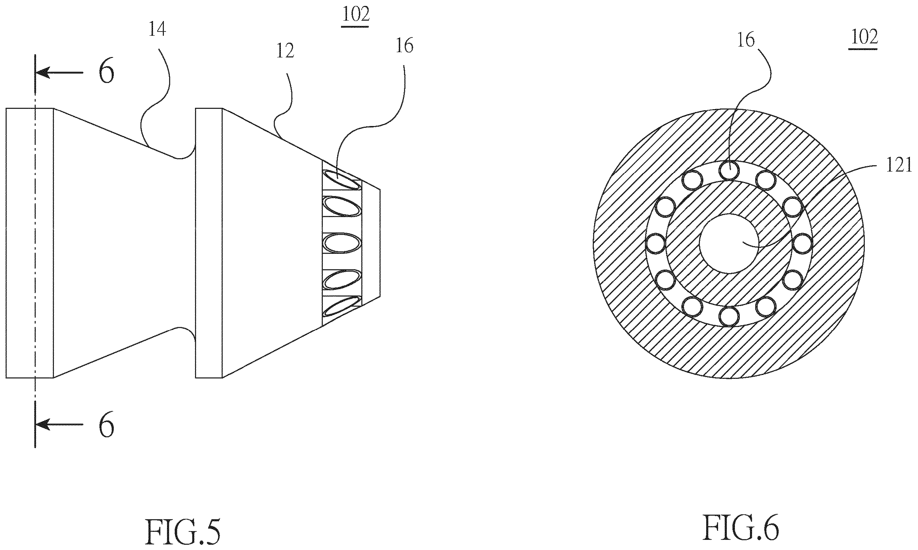

Referring to FIGS. 3 to 6, a combination bioactive silicate glass (e.g., bioactive glass) medicine carrier and shunt in accordance with a third preferred embodiment of the invention is shown and comprises a bullet shaped, capsule shaped, or olive shaped shunt 102 which is a capillary member made of bioactive silicate glass (e.g., bioactive glass). The shunt 102 includes a plurality of tunnel shaped medicine storages 16 equally spaced around an axial channel 121. The medicine storages 16 are used to store medicines such as anti-inflammatory drugs, anti-scarring drugs, anti-cancer drugs, or cell-activating drugs. Alternatively, the medicine storages 16 are used to direct flow if there is no medicine stored therein. The axial channel 121 is used to direct flow or store medicine. At least one direction check member 14 is provided on an outer surface of the shunt 102 and is adapted to prevent the shunt 102 from moving toward the insertion direction. Thus, the shunt 102 is stably disposed in a predetermined position. One end of the shunt 102 is formed as a tapered insertion end 12 so as to facilitate the shunt 102 to insert into the predetermined position.

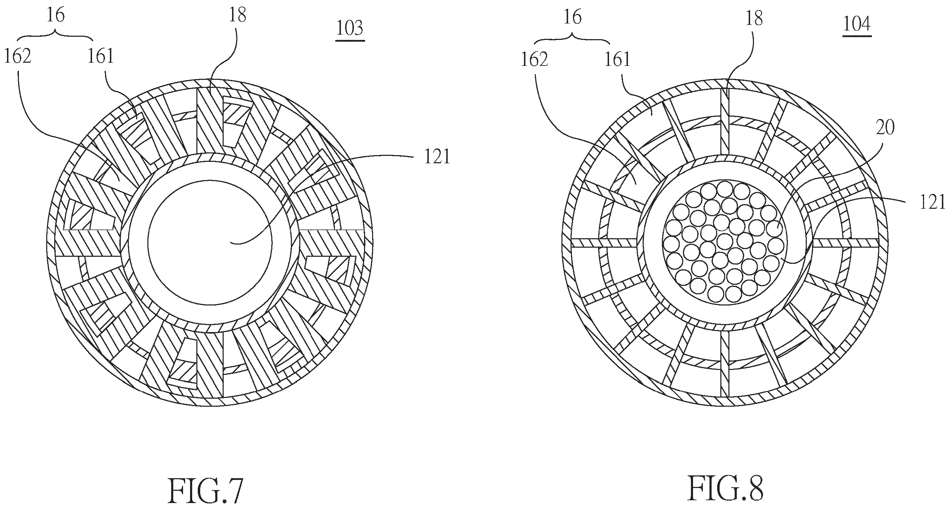

Referring to FIG. 7, a shunt 103 in accordance with a preferred embodiment of the invention is shown. Each of a plurality of medicine storages 16 of the distributor 103 includes at least one outer space 161 and at least one inner space 162. Medicine stored in the outer space 161 and the inner space 162 can be the same or different. A plurality of dividing members 18 are provided and each dividing member 18 is provided between any two adjacent ones of the outer space 161 and the inner space 162.

Thus, the medicine can be divided. The medicine storages 16 are arranged in a predetermined order rather than being coral shaped or irregular hole shaped. Alternatively, the medicine storages 16 have different thicknesses and are arranged in a predetermined order. As shown in FIG. 7, thicknesses of the dividing members 18 can be the same or different so as to take advantage of different biodegrading speeds to control time and amount of medicine being discharged. In the embodiment, the dividing members 18 are arranged in an order of being thick, thin, thick, thin, etc.

Referring to FIG. 8, a distributor 104 in accordance with a preferred embodiment of the invention is shown and includes a plurality of axial non-parallel hollow fiber strands 20 made of bioactive silicate. The fiber strands 20 have a very small diameter. Thus, high pressure fluid at one end of the distributor 104 may flow to the low pressure other end thereof through the fiber strands 20 due to capillary action of the fiber bundle 20. The flow will stop when pressures at two ends of the distributor 104 are equal.

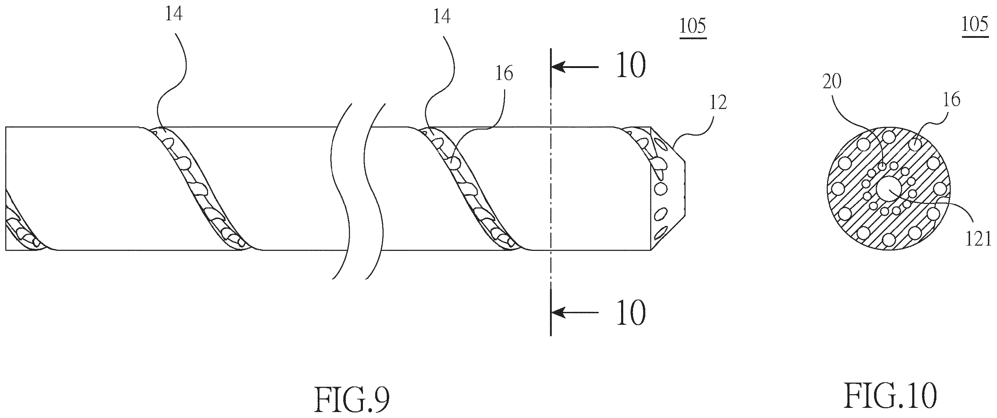

Referring to FIGS. 9 and 10, a shunt 105 in accordance with a preferred embodiment of the invention is shown and includes a plurality of tunnel shaped parallel medicine storages 16 open to an insertion end 12 at one end. The medicine storage 16 has a circular cross-section. The shunt 105 further comprises a plurality of groove shaped direction check members 14 on an intermediate portion of an outer surface of the distributor 105 for increasing positioning and performance of the distributor 105.

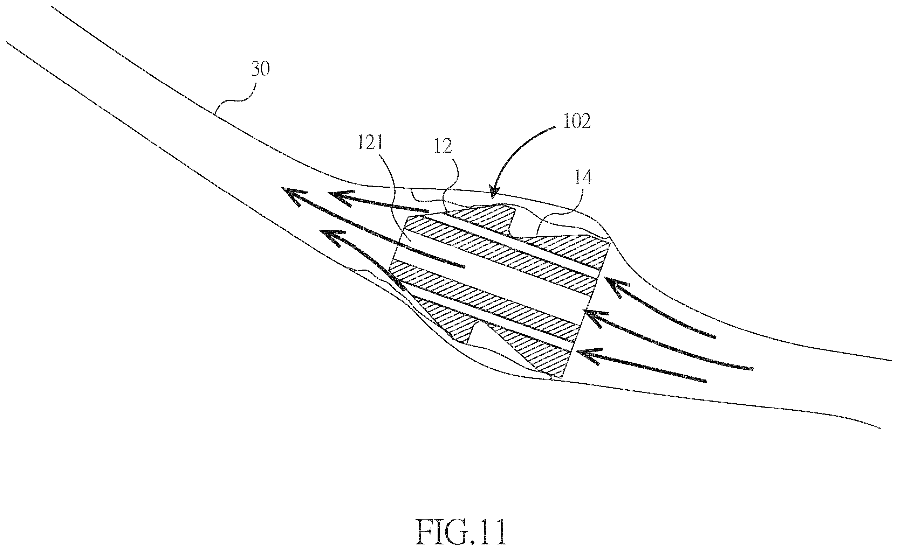

Referring to FIG. 11 in conjunction with FIGS. 3 to 6, the shunt 102 is inserted into a blood vessel 30 as support. Further, blood may flow from one end of the shunt 102 to the other end thereof through the channel 121 (or the medicine storages 16). While not shown, the medicine storages 16 are adapted to store medicine such as anti-inflammatory drugs, anti-scarring drugs, anti-cancer drugs, or cell-activating drugs. After the shunt 102 has been inserted into a predetermined position of the blood vessel, a small amount of medicine is sufficient to heal a wound since it is possible of directly discharging the medicine in the blood vessel proximate the location of the wound (or a target).

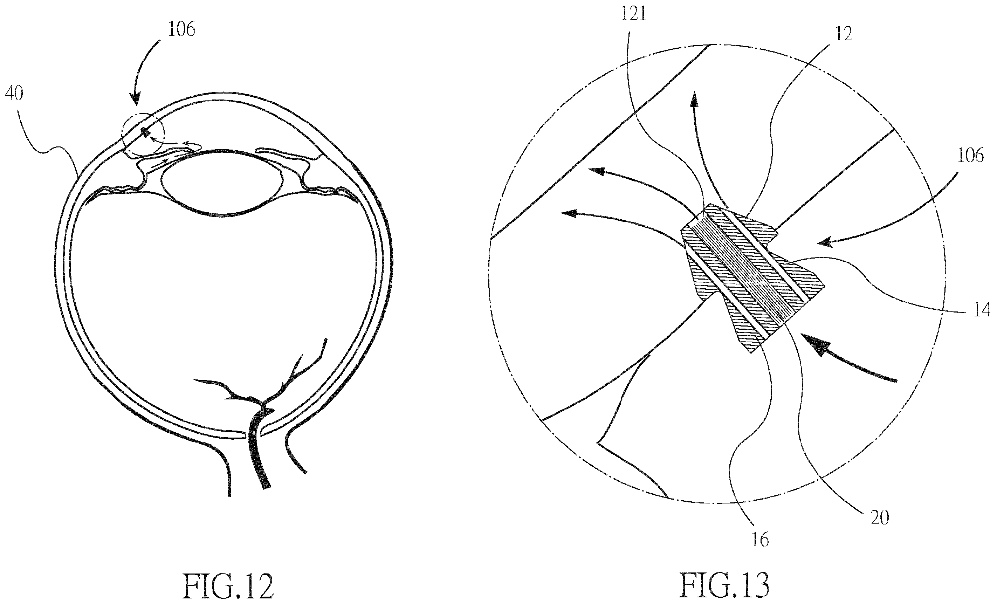

Referring to FIGS. 12 and 13 in conjunction with FIGS. 3 to 6, a shunt 106 in accordance with a preferred embodiment of the invention is shown. The shunt 106 is provided in an inner surface of an eyeball 40. The shunt 106 is substantially the same as the shunt 102 of FIG. 3 except the following: A fiber bundle 20 is provided in the axial channel 121 of the shunt 106. Fluid in the front chamber of the eyeball 40 may be discharged through the fiber bundle 20 due to capillary action of the fiber bundle 20, thereby lowering eye pressure. The medicine storages 16 are adapted to direct flow if they are not blocked by medicine.

The bio-activated glass may be degraded, from inside to outside, in 24 hours, one month, six months or one to three years. The time required for degradation depends on sizes, wall thicknesses, lengths and layers of the shunts 10, 101, 102, 103, 104, 105 and 106. The bioactive silicate (e.g., bioactive glass) having different compositions or a composition of high density silica can carry out the different times of degradation. As a result, time and amount of medicine being discharged can be controlled.

The biosilicates are bioresorbable and water soluble.

## Claims

1. A combination of a bioactive silicate glass medicine carrier and shunt, comprising:
a shunt which is a micro capillary tubular member made of bioactive silicate glass, the shunt including an insertion end at a first end; wherein the shunt further comprises an axial channel;
wherein gaps in the bioactive silicate glass serve as at least one medicine storage for storing medicines.

2. The combination of a bioactive silicate glass medicine carrier and shunt of claim 1, further comprising a fiber bundle disposed through the axial channel.

3. A combination of a bioactive silicate glass medicine carrier and shunt according to claim 1, wherein
the shunt including at least one direction check member disposed on an outer surface, and a tapered insertion end at the first end;
wherein gaps in the bioactive silicate glass define a plurality of tunnel-shaped medicine storages equally spaced around the axial channel for storing medicines.

4. The combination of a bioactive silicate glass medicine carrier and shunt of claim 3, wherein the shunt further comprises a plurality of groove shaped direction check members on an intermediate portion of an outer surface.

5. The combination of a bioactive silicate glass medicine carrier and shunt of claim 3, wherein the medicine storages are arranged in a predetermined order or have different thicknesses and are arranged in a predetermined order.

6. The combination of a bioactive silicate glass medicine carrier and shunt of claim 5, wherein each of the medicine storages includes at least one outer space, at least one inner space and a plurality of dividing members each disposed between any two adjacent ones of the outer space and the inner space; and wherein the dividing members have the same or different thicknesses so as to take advantage of different biodegrading speeds to control time and amount of medicine being discharged.

7. The combination of a bioactive silicate glass medicine carrier and shunt of claim 3, wherein the shunt is shaped as a bullet, a capsule, or an olive.

## Patentansprüche

1. Kombination aus einem bioaktiven Silikatglas-Medikamententräger und Shunt, welche umfasst:
einen Shunt, der ein mikrokapillares röhrenförmiges Element ist, das aus bioaktivem Silikatglas gefertigt ist, wobei der Shunt ein Einführungsende an einem ersten Ende aufweist; wobei der Shunt ferner einen axialen Kanal umfasst;
wobei Lücken in dem bioaktiven Silikatglas als mindestens ein Medikamentenspeicher zum Speichern von Medikamenten dienen.

2. Kombination aus einem bioaktiven Silikatglas-Medikamententräger und Shunt nach Anspruch 1, welche ferner ein Faserbündel umfasst, das durch den axialen Kanal angeordnet ist.

3. Kombination aus einem bioaktiven Silikatglas-Medikamententräger und Shunt nach Anspruch 1, bei welcher
der Shunt mindestens ein Richtungsprüfungselement, das an einer Außenfläche angeordnet ist, und ein sich verjüngendes Einführungsende am ersten Ende umfasst;
wobei Lücken in dem bioaktiven Silikatglas eine Vielzahl von tunnelförmigen Medikamentenspeichern definieren, die in gleichen Abständen um den axialen Kanal herum angeordnet sind, um Medikamente zu speichern.

4. Kombination aus einem bioaktiven Silikatglas-Medikamententräger und Shunt nach Anspruch 3, bei welcher der Shunt ferner eine Vielzahl von rillenförmigen Richtungsprüfungselementen an einem Zwischenabschnitt einer Außenfläche umfasst.

5. Kombination aus einem bioaktiven Silikatglas-Medikamententräger und Shunt nach Anspruch 3, bei welcher die Medikamentenspeicher in einer vorgegebenen Reihenfolge angeordnet sind oder unterschiedliche Dicken haben und in einer vorgegebenen Reihenfolge angeordnet sind.

6. Kombination aus einem bioaktiven Silikatglas-Medikamententräger und Shunt nach Anspruch 5, bei welcher jeder der Medikamentenspeicher mindestens einen äußeren Raum, mindestens einen inneren Raum und eine Vielzahl von Trennelementen umfasst, die jeweils zwischen zwei beliebigen benachbarten der äußeren Räume und inneren Räume angeordnet sind; und wobei die Trennelemente die gleichen oder unterschiedliche Dicken haben, um die unterschiedlichen biologischen Abbaugeschwindigkeiten auszunutzen und so den Zeitpunkt und die Menge der Medikamentenfreisetzung zu steuern.

7. Kombination aus einem bioaktiven Silikatglas-Medikamententräger und Shunt nach Anspruch 3, bei welcher der Shunt die Form einer Kugel, einer Kapsel oder einer Olive aufweist.

## Revendications

1. Combinaison d'un support de médicament et d'un shunt en verre de silicate bioactif, comprenant :
un shunt qui est un élément tubulaire microcapillaire en verre de silicate bioactif, le shunt incluant une extrémité d'insertion sur une première extrémité ; dans laquelle le shunt comprend en outre un canal axial ;
dans laquelle des interstices dans le verre de silicate bioactif servent d'au moins un stockage de médicaments pour stocker des médicaments.

2. Combinaison d'un support de médicament et d'un shunt en verre de silicate bioactif selon la revendication 1, comprenant en outre un faisceau de fibres disposé à travers le canal axial.

3. Combinaison d'un support de médicament et d'un shunt en verre de silicate bioactif selon la revendication 1, dans laquelle
le shunt incluant au moins un élément de contrôle de direction disposé sur une surface extérieure, et une extrémité d'insertion conique sur la première extrémité ;
dans laquelle des interstices dans le verre de silicate bioactif définissent une pluralité de stockages de médicaments en forme de tunnel équidistants autour du canal axial pour stocker des médicaments.

4. Combinaison d'un support de médicament et d'un shunt en verre de silicate bioactif selon la revendication 3, dans laquelle le shunt comprend en outre une pluralité d'éléments de contrôle de direction en forme de rainure sur une partie intermédiaire d'une surface extérieure.

5. Combinaison d'un support de médicament et d'un shunt en verre de silicate bioactif selon la revendication 3, dans laquelle les stockages de médicaments sont agencés dans un ordre prédéterminé ou présentent des épaisseurs différentes et sont agencés dans un ordre prédéterminé.

6. Combinaison d'un support de médicament et d'un shunt en verre de silicate bioactif selon la revendication 5, dans laquelle chacun des stockages de médicaments inclut au moins un espace extérieur, au moins un espace intérieur et une pluralité d'éléments de séparation disposés chacun entre deux espaces adjacents parmi l'espace extérieur et l'espace intérieur ; et dans laquelle les éléments de séparation présentent des épaisseurs identiques ou différentes de manière à tirer parti de différentes vitesses de biodégradation pour réguler le temps et la quantité de médicament distribué.

7. Combinaison d'un support de médicament et d'un shunt en verre de silicate bioactif selon la revendication 3, dans laquelle le shunt se présente sous forme de balle, de capsule ou d'olive.
